# EUROPEAN PATENT APPLICATION

(11) **EP 3 942 992 A1**
(43) Date of publication of application: **26.01.2022**
(21) Application number: 19934208.0
(22) Date of filing: 07.09.2019
(51) Int. Cl.: A61B 1/04

(54) **MAGNETIC CONTROL DEVICE OF CAPSULE ENDOSCOPE AND METHOD FOR CONTROLLING MOVEMENT OF CAPSULE ENDOSCOPE IN TISSUE CAVITY**

(30) Priority: 17.06.2019 CN 201910518998
(71) Applicant: Shenzhen Sibernetics Co., Ltd., Shenzhen, Guangdong 518000 (CN)
(72) Inventor: PENG, Can, Shenzhen, Guangdong 518000 (CN); LIU, Liu, Shenzhen, Guangdong 518000 (CN); XIA, Ran, Shenzhen, Guangdong 518000 (CN); XIA, Bin, Shenzhen, Guangdong 518000 (CN); ZHAO, Yu, Shenzhen, Guangdong 518000 (CN)
(74) Representative: Isern Patentes y Marcas S.L.
(86) International application number: PCT/CN2019/104815
(87) International publication number: WO 2020/252940

(57) **Abstract**

A magnetic control device (30) for a capsule endoscope (10), comprising at least a first magnet (11) and an imaging device (12), and the capsule endoscope (10) is located in a tissue cavity (3); wherein the magnetic control device (30) comprises a second magnet (31) and a first induction coil (32) arranged around the second magnet (31), the magnetic control device (30) is configured to generate a driving force to the capsule endoscope (10) to move the capsule endoscope (10) from a first position (PI) in the tissue cavity (3) to a second position towards an opposite side in the tissue cavity (3), control a variable magnetic field to decelerate the capsule endoscope (10) to a predetermined speed when the capsule endoscope (10) approaches the second position (P2), and the magnetic control device (30) is configured to generate the variable magnetic field for the capsule endoscope (10). In this case, the moving path of the capsule endoscope (10) in the tissue cavity (3) can be reasonably optimized, which make it possible to comprehensively inspect the tissue cavity (3) and protect the inner wall of the tissue cavity (3) as well.

## Description

### BACKGROUND OF INVENTION

### Field of Invention

The disclosure relates generally to a magnetic control device for a capsule endoscope and a method for controlling movement of the capsule endoscope in a tissue cavity.

### Description of Related Art

With the development of modern medical technology, lesions on tissue walls of digestive tracts such as stomach, large intestine, small intestine and the like can be snooped by swallowing a capsule endoscope which can help doctors to obtain accurate information of lesion areas in the digestive tracts, so as to assist them in diagnosing and treating patients. Such capsule endoscopes generally include a magnet controlled by an external magnetic control device, an imaging device, and a wireless transmission device that transmits captured images to the outside. Specifically, a doctor, a nurse or other operators magnetically guide a capsule-type endoscope located in an organ of a tissue cavity such as stomach, small intestine and the like by controlling the external magnetic control device, so that the capsule-type endoscope moves inside the tissue cavity and captures an image of a specific position (such as a lesion area) inside the tissue cavity, then transmits the captured image to an external display device through wireless transmission and the like. The doctor or the like can observe and diagnose the digestive tract of the patient via the display device.

In the process of guiding the capsule endoscope in the tissue cavity based on the magnetic control method and device, the movement of the capsule endoscope in the tissue cavity should be as simple and easy to implement as possible in order to facilitate the operation and make the capsule endoscope easy to control and able to clearly capture possible lesions in the tissue cavity. For example, the magnetic control method commonly used at present is to roll the capsule endoscope at the bottom of the stomach or large intestine for photography. However, since the imaging device of the capsule endoscope takes a picture against a tissue wall, such as the stomach wall, the field angle of the image captured by the capsule endoscope is limited, as a result the spatial position of the image taken in the tissue cavity is insufficient and there may be a case of missed detection.

Therefore, it is necessary to improve the above conventional magnetic control method and device in order to give full play to the image capturing ability of the capsule endoscope in the tissue cavity, especially in the cavity with large space such as the stomach or the large intestine, and reduce the possibility of missed detection.

### SUMMARY OF INVENTION

In view of the above existing situations, the objective of the present invention is to provide a magnetic control device for a capsule endoscope capable of improving a moving path and an image capturing manner of a capsule endoscope in a tissue cavity so as to cover the tissue cavity.

To achieve the objective, a first aspect of the disclosure provides a magnetic control device for a capsule endoscope, wherein the capsule endoscope comprises at least a first magnet and an imaging device, and is located in a tissue cavity; the magnetic control device comprises a second magnet and a first induction coil arranged around the second magnet, and the magnetic control device is configured to generate a driving force to the capsule endoscope to move the capsule endoscope from a first position in the tissue cavity to a second position towards an opposite side in the tissue cavity; a variable magnetic field is controlled to decelerate the capsule endoscope to a predetermined speed when the capsule endoscope approaches the second position, the magnetic control device is configured to generate the variable magnetic field for the capsule endoscope, and the variable magnetic field comprises a base magnetic field generated by the second magnet and an induced magnetic field generated by the first induction coil; and the direction of a magnetic axis of the first induction coil is kept in a fixed orientation, and the variable magnetic field is generated by changing at least one of the relative position of a magnetic pole of the second magnet relative to the first magnet, the current magnitude of the first induction coil, and the current direction of the first induction coil.

In the first aspect of the disclosure, the movement of the capsule endoscope is controlled by configuring the magnetic control device, so that the path of the capsule endoscope in the tissue cavity can be optimized, and the velocity of the capsule endoscope can be controlled; and the examination coverage rate of the tissue cavity can be improved, and the damage to the tissue cavity caused by the overspeed of the capsule endoscope can be inhibited.

In addition, in the magnetic control device for the capsule endoscope according to the first aspect of the disclosure, optionally, the magnetic control device is configured to control the variable magnetic field to accelerate the capsule endoscope when the capsule endoscope is in the first position, and to control the variable magnetic field to decelerate the capsule endoscope to the predetermined speed when the capsule endoscope approaches the second position, during the movement of the capsule endoscope from the first position to the second position. Thereby, the moving path of the capsule endoscope in the tissue cavity can be flexibly controlled by changing the velocity of the capsule endoscope.

In addition, in the magnetic control device for the capsule endoscope according to the first aspect of the disclosure, optionally, the magnetic control device is configured to control the variable magnetic field to accelerate the capsule endoscope to an initial speed and move a predetermined distance while maintaining the initial speed when the capsule endoscope is in the first position, then to control the variable magnetic field to decelerate the capsule endoscope to the predetermined speed when the capsule endoscope approaches the second position, during the movement of the capsule endoscope from the first position to the second position. Thereby, the buffering speed when the capsule endoscope decelerates can be reasonably controlled, so that the damage of the capsule endoscope to the side wall of the tissue cavity and the like is further inhibited.

In addition, in the magnetic control device for the capsule endoscope according to the first aspect of the disclosure, optionally, the capsule endoscope shoots the inside of the tissue cavity facing a side of the first position when the capsule endoscope is located in the second position. Thereby, when the capsule endoscope reaches a proper position, shooting the tissue cavity can be realized with the imaging device with a higher freedom degree at the other side of the capsule endoscope, and the coverage area captured by the imaging device can be improved.

In addition, in the magnetic control device for the capsule endoscope according to the first aspect of the disclosure, optionally, the first position is a side wall on one side in the tissue cavity, and the second position is a side wall at the other side in the tissue cavity. Thereby, the capsule endoscope can be stabilized by means of the supporting force of the side wall of the tissue cavity, so that the capsule endoscope can shoot more stably under the control of the variable magnetic field.

In addition, in the magnetic control device for the capsule endoscope according to the first aspect of the disclosure, the process of moving the capsule endoscope from the first position in the tissue cavity to the second position in the tissue cavity and back again to a third position in the tissue cavity is a shuttle process, wherein the first position and the third position are located on one side in the tissue cavity, and the second position is located at the other side in the tissue cavity; and the magnetic control device is configured to control the variable magnetic field to deviate the capsule endoscope during the shuttle process, thereby deviating the third position of the capsule endoscope from the first position. Thereby, a comprehensive inspection to the tissue cavity can be realized by the shuttle mode, and a missed inspection to pathological changes of the tissue cavity can be avoided.

In addition, in the magnetic control device for the capsule endoscope according to the first aspect of the disclosure, optionally, the magnetic control device is configured to move the tissue cavity in a direction forming an included angle with the direction of the magnetic axis to deviate the position of the first magnet from the magnetic axis when the capsule endoscope is in the first position or the second position in the tissue cavity, and the second magnet generates a magnetic force acting on the capsule endoscope in the direction of the magnetic axis. Thereby, the movement control of the capsule endoscope can be realized by adjusting and controlling the second magnet.

A second aspect of the disclosure provides a magnetic control device for a capsule endoscope, wherein the capsule endoscope comprises at least a first magnet and an imaging device, and the capsule endoscope is located in a tissue cavity; the magnetic control device comprises a second magnet and a first induction coil arranged around the second magnet, the magnetic control device is configured to generate a variable magnetic field for the capsule endoscope, and the variable magnetic field comprises a base magnetic field generated by the second magnet and an induced magnetic field generated by the first induction coil; and the direction of the magnetic axis of the first induction coil is kept in a fixed orientation, and the variable magnetic field is generated by changing at least one of the relative position of a magnetic pole of the second magnet relative to the first magnet, the current magnitude of the first induction coil, and the current direction of the first induction coil.

In the second aspect of the disclosure, the movement of the capsule endoscope is controlled by configuring the magnetic control device, so that the path of the capsule endoscope in the tissue cavity can be optimized, the velocity of the capsule endoscope can be controlled, and the examination coverage rate of the tissue cavity can be improved.

In addition, in the magnetic control device for the capsule endoscope according to the second aspect of the disclosure, optionally, the magnetic control device is configured such that during the movement of the capsule endoscope from the first position to the second position, the second magnet generates a first magnetic force to the capsule endoscope and the first induction coil generates a second magnetic force to the capsule endoscope; and a deviating movement is generated to the capsule endoscope by forming an included angle between a magnetic force direction of the first magnetic force and a magnetic force direction of the second magnetic force. Thereby, the capsule endoscope can be driven to move to a specific path conveniently by the cooperation of the base magnetic field and the induced magnetic field.

In addition, in the magnetic control device for the capsule endoscope according to the second aspect of the disclosure, optionally, a fluid is contained in the tissue cavity and a liquid surface is formed in the tissue cavity, and the magnetic control device is configured to control the variable magnetic field such that the capsule endoscope is at the liquid surface and remains at a position relative to the tissue cavity, and the tissue cavity is moved and shot inside. Thereby, the capsule endoscope can stably shoot at the liquid surface position with the buoyancy of the liquid, the gravity of the capsule endoscope and the magnetic force of the variable magnetic field, and the tissue cavity can be shot more conveniently by adjusting the liquid surface position and moving the capsule endoscope at the liquid surface.

In addition, in the magnetic control device for the capsule endoscope according to the second aspect of the disclosure, optionally, a liquid is contained in the tissue cavity and a liquid surface is formed in the tissue cavity, and the height of the liquid surface is larger than the length of the capsule endoscope. Thereby, the capsule endoscope can be more conveniently controlled by the buoyancy of the liquid.

In addition, in the magnetic control device for the capsule endoscope according to the second aspect of the disclosure, optionally, the tissue cavity is moved relative to the capsule endoscope to shoot the inside of the tissue cavity. Thereby, more stable control over the capsule endoscope can be achieved.

In addition, in the magnetic control device for the capsule endoscope according to the second aspect of the disclosure, optionally, the first induction coil is arranged on the same side as the second magnet. Thereby, the magnetic field force generated by the first induction coil and the magnetic field force generated by the second magnet can be more concentrated, and the capsule endoscope can be more easily controlled.

In addition, in the magnetic control device for the capsule endoscope according to the second aspect of the disclosure, optionally, the magnetic control device further comprises a second induction coil arranged at a different side from the first induction coil, and the second induction coil is arranged at a different side from the second magnet. Thereby, the capsule endoscope in the tissue cavity can move more stably under the action of the first induction coil, the second induction coil and the second magnet which are positioned at different sides of the tissue cavity.

In addition, in the magnetic control device for the capsule endoscope according to the second aspect of the disclosure, optionally, the magnetic control device further comprises a third induction coil that generates a magnetic force acting on the capsule endoscope to position the capsule endoscope at a predetermined position within the tissue cavity. Thereby, the capsule endoscope can be positioned in a required working area more accurately.

In addition, in the magnetic control device for the capsule endoscope according to the second aspect of the disclosure, optionally, the diameter of the third induction coil is smaller than the diameter of the first induction coil and the diameter of the second induction coil. Thereby, the positioning of the capsule endoscope in the tissue cavity can be more easily realized by the third induction coil which is in a same order of magnitude as the size of the capsule endoscope and is smaller than the first induction coil and the second induction coil.

In addition, in the magnetic control device for the capsule endoscope according to the second aspect of the disclosure, optionally, the third induction coil is arranged on the same side as the second induction coil. Thereby, the positioning of the capsule endoscope in the tissue cavity can be more easily realized.

Further, in the magnetic control device for the capsule endoscope according to the second aspect of the disclosure, optionally, an imaging device is provided at both ends of the capsule endoscope, respectively. Thereby, the rotation angle of the capsule endoscope can be reduced, and the tissue cavity can be shot more easily and omni-directionally.

Further, in the magnetic control device for the capsule endoscope according to the second aspect of the disclosure, optionally, the center of gravity of the capsule endoscope is closer to the other end with respect to one end. Thereby, the capsule endoscope can shoot at a predetermined position in the tissue cavity more stably.

In addition, in the magnetic control device for the capsule endoscope according to the second aspect of the disclosure, optionally, the magnetic control device is configured to adjust the orientation of the imaging device at the other end of the capsule endoscope with one end of the capsule endoscope located at the side wall in the tissue cavity being as a fulcrum by controlling the variable magnetic field when the capsule endoscope is located at the side wall in the tissue cavity. Thereby, the capsule endoscope can be stably positioned on the inner side wall of the tissue cavity by changing the variable magnetic field by means of the supporting force of the inner side wall of the tissue cavity.

In addition, in the magnetic control device for the capsule endoscope according to the second aspect of the disclosure, optionally, the magnetic control device is configured to adjust the orientation of the imaging device at the other end of the capsule endoscope and shoot, with one end of the capsule endoscope located at the side wall in the tissue cavity being as a fulcrum, by controlling the variable magnetic field. Thereby, the capsule endoscope imaging device can be adjusted in the orientation by changing the variable magnetic field to stably shoot the other side in the tissue cavity.

In addition, in the magnetic control device for the capsule endoscope according to the second aspect of the disclosure, optionally, the magnetic control device is configured to control the variable magnetic field by changing a position of a magnetic pole of the second magnet relative to the first magnet. Thereby, the imaging device of the capsule endoscope can be conveniently adjusted to shoot the tissue cavity.

In addition, in the magnetic control device for the capsule endoscope according to the second aspect of the disclosure, optionally, the imaging device of the capsule endoscope performs automatic shooting in the tissue cavity at regular time intervals. Thereby, the capsule endoscope can be activated before or after the capsule endoscope enters the tissue cavity , so that the capsule endoscope can automatically shoot in the tissue cavity, and a more comprehensive image of the tissue cavity can be shot by the capsule endoscope.

Further, in the magnetic control device for the capsule endoscope according to the second aspect of the disclosure, optionally, the direction of the magnetic axis is a vertical direction, and the magnetic axis passes through the second magnet.

In addition, in the magnetic control device for the capsule endoscope according to the second aspect of the disclosure, optionally, the second magnet is a cylinder. Thereby, the second magnet can be operated more conveniently.

In addition, in the magnetic control device for the capsule endoscope according to the second aspect of the disclosure, optionally, the second magnet is arranged around the first induction coil in such a manner that the second magnet is rotatable around a point as the center at which the second magnet intersects the magnetic axis of the first induction coil. Thereby, the path of the capsule endoscope can be optimized by adjusting the direction and the magnitude of the magnetic force of the first induction coil and assisting the second magnet for controlling the movement direction of the capsule endoscope and the deviation of the lens.

In addition, in the magnetic control device for the capsule endoscope according to the second aspect of the disclosure, optionally, a positioning place of the capsule endoscope relative to the tissue cavity is obtained by respectively detecting magnetic fields of the first magnet, the second magnet, the first induction coil using a magnetic sensor array, and calculating a position of the magnetic sensor array relative to the capsule endoscope based on a model of a magnetic dipole of the capsule endoscope. Thereby, the position of the capsule endoscope in the tissue cavity can be accurately positioned.

In addition, in the magnetic control device for the capsule endoscope according to the second aspect of the disclosure, optionally, a moving path of the capsule endoscope in the tissue cavity is planned and adjusted based on the positioning place, so that a shooting region of the capsule endoscope substantially covers the tissue cavity. Thereby, a next moving path of the capsule endoscope can be reasonably optimized, and the capsule endoscope can realize omni-directional shooting of the tissue cavity.

In addition, in the magnetic control device for the capsule endoscope according to the second aspect of the disclosure, optionally, the tissue cavity is a stomach. As a result, the relatively large space of the stomach can facilitate the movement and the path selection of the capsule endoscope.

In addition, in the magnetic control device for the capsule endoscope according to the second aspect of the disclosure, optionally, the process of moving the capsule endoscope from the first position of the stomach to the second position of the stomach and back again to the third position of the stomach is a shuttle process, wherein the first position and the third position are located on one side of the stomach, and the second position is located at the other side of the stomach; during the shuttle process, the capsule endoscope is further moved to a fourth position other than the first position and the second position before the capsule endoscope moves to the second position of the stomach, wherein the fourth position is not located in a plane formed by the first position, the second position and the third position. Thereby, all parts of the tissue cavity can be comprehensively shot.

In addition, in the magnetic control device for the capsule endoscope according to the second aspect of the disclosure, optionally, during the shuttle process, the capsule endoscope is further moved to a fifth position between the second position and the third position before the capsule endoscope is moved to the third position of the stomach, wherein the fifth position is not located in a plane formed by the first position, the second position and the third position. Thereby, all parts of the tissue cavity can be comprehensively shot.

In addition, in the magnetic control device for the capsule endoscope according to the second aspect of the disclosure, optionally, the first position, the second position, the third position, the fourth position, and the fifth position are all located at the inner wall of the stomach. Thereby, the capsule endoscope can shoot more stably by the aid of the supporting force of the inner wall of the stomach, and all parts of the tissue cavity can be shot comprehensively.

A third aspect of the disclosure provides a method for moving a capsule endoscope which comprises at least a first magnet and an imaging device in a tissue cavity,, comprising: enabling the capsule endoscope to enter the tissue cavity; generating a variable magnetic field for the capsule endoscope, and generating a driving force to the capsule endoscope by controlling the variable magnetic field, thereby moving the capsule endoscope from a first position in the tissue cavity to a second position towards an opposite side in the tissue cavity; and controlling the variable magnetic field to decelerate the capsule endoscope to a predetermined speed when the capsule endoscope approaches the second position, wherein the variable magnetic field comprises a base magnetic field generated by a second magnet and an induced magnetic field generated by a first induction coil; and the direction of the magnetic axis of the first induction coil is kept in a fixed orientation, and the variable magnetic field is changed by changing at least one of the relative position of a magnetic pole of the second magnet with respect to the first magnet, the current magnitude of the first induction coil, and the current direction of the first induction coil.

In the third aspect of the disclosure, the movement of the capsule endoscope is controlled by the variable magnetic field, so that the path of the capsule endoscope in the tissue cavity can be optimized, and the velocity of the capsule endoscope can be controlled; and the examination coverage rate of the tissue cavity can be improved, and the damage to the tissue cavity caused by the overspeed of the capsule endoscope can be inhibited.

In addition, in the method for controlling the movement of a capsule endoscope in a tissue cavity according to the third aspect of the disclosure, it optionally comprises controlling the variable magnetic field to accelerate the capsule endoscope to an initial speed and move it for a predetermined distance maintaining the initial speed when the capsule endoscope is in the first position, then controlling the variable magnetic field to decelerate the capsule endoscope to the predetermined speed when the capsule endoscope approaches the second position, during the movement of the capsule endoscope from the first position to the second position. Thereby, the moving path of the capsule endoscope in the tissue cavity can be flexibly controlled by changing the velocity of the capsule endoscope.

In addition, in a method for controlling the movement of the capsule endoscope in the tissue cavity according to the third aspect of the disclosure, it optionally comprises controlling the variable magnetic field to accelerate the capsule endoscope to an initial speed and move it for a predetermined distance maintaining the initial speed when the capsule endoscope is in the first position, then controlling the variable magnetic field to decelerate the capsule endoscope to the predetermined speed when the capsule endoscope approaches the second position, during the movement of the capsule endoscope from the first position to the second position. Thereby, the buffering speed when the capsule endoscope decelerates can be reasonably controlled, so that the damage of the capsule endoscope to the side wall of the tissue cavity and the like is further inhibited.

In addition, in the method for controlling the movement of the capsule endoscope in the tissue cavity according to the third aspect of the disclosure, optionally, the capsule endoscope shoots the inside of the tissue cavity with a side of the capsule endoscope facing the first position when the capsule endoscope is located in the second position. Thereby, when the capsule endoscope reaches a proper position, shooting the tissue cavity can be realized by the imaging device with higher freedom degree at the other side of the capsule endoscope, and the shooting coverage area of the imaging device can be improved.

In addition, in the method for controlling movement of a capsule endoscope within a tissue cavity according to a third aspect of the disclosure, optionally, the first position is a side wall on one side in the tissue cavity, and the second position is a side wall at the other side in the tissue cavity. Thereby, the capsule endoscope can be stabilized by means of the supporting force of the side wall of the tissue cavity, so that the capsule endoscope can shoot more stably under the control of the variable magnetic field.

In addition, in the method for controlling the movement of the capsule endoscope in the tissue cavity according to the third aspect of the disclosure, optionally, the process of moving the capsule endoscope from the first position in the tissue cavity to the second position in the tissue cavity and back again to a third position in the tissue cavity is a shuttle process, wherein the first position and the third position are located on one side in the tissue cavity, and the second position is located at the other side in the tissue cavity; and the variable magnetic field is controlled to deviate the capsule endoscope during the shuttle process, thereby deviating the third position of the capsule endoscope from the first position. Thereby, a comprehensive inspection to the tissue cavity can be realized by the shuttle mode, and a missed inspection to pathological changes of the tissue cavity can be avoided.

In addition, in the method for controlling the movement of the capsule endoscope in the tissue cavity according to the third aspect of the disclosure, optionally, the tissue cavity is moved in a direction forming an included angle with the direction of the magnetic axis to deviate the position of the first magnet from the magnetic axis when the capsule endoscope is in the first position or the second position in the tissue cavity, and the second magnet generates a magnetic force acting on the capsule endoscope in the direction of the magnetic axis. Thereby, the movement control of the capsule endoscope can be realized by adjusting and controlling the second magnet.

A fourth aspect of the disclosure provides a method for controlling movement of a capsule endoscope comprising at least a first magnet and an imaging device in a tissue cavity, comprising: enabling the capsule endoscope to enter the tissue cavity; and generating a variable magnetic field for the capsule endoscope, and generating a driving force to the capsule endoscope by controlling the variable magnetic field, wherein the variable magnetic field comprises a base magnetic field generated by a second magnet and an induction magnetic field generated by a first induction coil; and the direction of a magnetic axis of the first induction coil is kept in a fixed orientation, and the variable magnetic field is changed by changing at least one of the relative position of a magnetic pole of the second magnet relative to the first magnet, the current magnitude of the first induction coil, and the current direction of the first induction coil.

In the fourth aspect of the disclosure, the movement of the capsule endoscope is controlled by a variable magnetic field, so that the path of the capsule endoscope in the tissue cavity can be optimized, the velocity of the capsule endoscope can be controlled, and the examination coverage rate of the tissue cavity can be improved.

In addition, in the method for controlling the movement of the capsule endoscope in the tissue cavity according to the fourth aspect of the disclosure, optionally, during movement of the capsule endoscope from the first position to the second position, the base magnetic field generates a first magnetic force to the capsule endoscope and the induced magnetic field generates a second magnetic force to the capsule endoscope; and a deviating movement is generated to the capsule endoscope by forming an included angle between a magnetic force direction of the first magnetic force and a magnetic force direction of the second magnetic force. Thereby, the capsule endoscope can be driven to move to a specific path conveniently by the cooperation of the base magnetic field and the induced magnetic field.

In addition, in the method for controlling movement of the capsule endoscope in the tissue cavity according to the fourth aspect of the disclosure, optionally, fluid is contained in the tissue cavity and a liquid surface is formed in the tissue cavity, and the variable magnetic field is controlled so that the capsule endoscope is at the liquid surface and remains at a position relative to the tissue cavity, and the tissue cavity is moved and shot inside. Thereby, the capsule endoscope can stably shoot at the liquid surface position by utilizing the buoyancy of the liquid, the gravity of the capsule endoscope and the magnetic force of the variable magnetic field, and the tissue cavity can be shot more conveniently by adjusting the liquid surface position and moving the capsule endoscope at the liquid surface.

In addition, in the method for controlling the movement of the capsule endoscope in the tissue cavity according to the fourth aspect of the disclosure, optionally, the first induction coil is arranged on the same side as the second magnet. Thereby, the magnetic field force generated by the first induction coil and the magnetic field force generated by the second magnet can be more concentrated, and the capsule endoscope can be more easily controlled.

In addition, in the method for controlling the movement of the capsule endoscope in the tissue cavity according to a fourth aspect of the disclosure, optionally, the magnetic control device further comprises a second induction coil arranged at a different side from the first induction coil, and the second induction coil is arranged at a different side from the second magnet. Thereby, the capsule endoscope in the tissue cavity can move more stably under the action of the first induction coil, the second induction coil and the second magnet which are positioned at different sides of the tissue cavity.

In addition, in the method for controlling the movement of the capsule endoscope in the tissue cavity according to a fourth aspect of the disclosure, optionally, the magnetic control device further comprises a third induction coil that generates a magnetic force acting on the capsule endoscope to position the capsule endoscope at a predetermined position within the tissue cavity.

In addition, in the method for controlling the movement of the capsule endoscope in the tissue cavity according to a fourth aspect of the disclosure, optionally, the diameter of the third induction coil is smaller than the diameter of the first induction coil and the diameter of the second induction coil. Thereby, the capsule endoscope can be positioned at a required working area more accurately.

In addition, in the method for controlling the movement of the capsule endoscope in the tissue cavity according to the fourth aspect of the disclosure, optionally, the third induction coil is arranged on the same side as the second induction coil. Thereby, the positioning of the capsule endoscope in the tissue cavity can be more easily realized by the third induction coil which is in a same order of magnitude as the size of the capsule endoscope and is smaller than the first induction coil and the second induction coil.

In addition, in the method for controlling the movement of the capsule endoscope in the tissue cavity according to the fourth aspect of the disclosure, optionally, the orientation of the imaging device is adjusted at the other end of the capsule endoscope with one end of the capsule endoscope located at the side wall in the tissue cavity being as a fulcrum by controlling the variable magnetic field, when the capsule endoscope is located at the side wall in the tissue cavity. Thus, the capsule endoscope can be stably positioned on the inner side wall of the tissue cavity by changing the variable magnetic field by means of the supporting force of the inner side wall of the tissue cavity.

In addition, in the method for controlling the movement of the capsule endoscope in the tissue cavity according to the fourth aspect of the disclosure, optionally, the direction of the magnetic axis is a vertical direction, and the magnetic axis passes through the second magnet. Thus, the capsule endoscope can be stabilized on the magnetic axis.

In addition, in the method for controlling the movement of the capsule endoscope in the tissue cavity according to the fourth aspect of the disclosure, optionally, the second magnet is arranged around the first induction coil in such a manner that the second magnet is rotatable around a point as the center at which the second magnet and the magnetic axis of the first induction coil intersect. Thereby, the path of the capsule endoscope can be optimized by adjusting the direction and the magnitude of the magnetic force of the induction coil and assisting the second magnet to control the moving direction of the capsule endoscope and the deviation of the lens.

In addition, in the method for controlling the movement of the capsule endoscope in the tissue cavity according to the fourth aspect of the disclosure, optionally, the process of moving the capsule endoscope from the first position of the tissue cavity to the second position of the tissue cavity and back again to the third position of the tissue cavity is a shuttle process, wherein the first position and the third position are located on one side of the tissue cavity, and the second position is located at the other side of the tissue cavity; during the shuttle process, the capsule endoscope is further moved to a fourth position other than the first position and the second position before the capsule endoscope moves to the second position of the tissue cavity, wherein the fourth position is not located in a plane formed by the first position, the second position and the third position. Thereby, all parts of the tissue cavity can be comprehensively shot.

In addition, in the method for controlling the movement of the capsule endoscope in the tissue cavity according to the fourth aspect of the disclosure, optionally, during the shuttle process, the capsule endoscope is further moved to a fifth position other than the second position and the third position before the capsule endoscope is moved to the third position of the tissue cavity, wherein the fifth position is not located in a plane formed by the first position, the second position and the third position. Thereby, all parts of the tissue cavity can be comprehensively shot.

In addition, in the method for controlling the movement of the capsule endoscope in the tissue cavity according to the fourth aspect of the disclosure, optionally, the first position, the second position, the third position, the fourth position, and the fifth position are all located at the inner wall of the tissue cavity. Thereby, the capsule endoscope can shoot more stably by the aid of the supporting force of the inner wall of the stomach, and all parts of the tissue cavity can be shot comprehensively.

According to the disclosure, it provides a method for controlling movement of a capsule endoscope in a tissue cavity that can improve the moving path and the image capturing manner of the capsule endoscope in the tissue cavity to enable the covering all over the tissue cavity.

### BRIEF DESCRIPTION OF THE DRAWINGS

Embodiments of the disclosure will now be explained in further detail, by way of example only, with reference to the accompanying drawings, in which:
Fig. 1 is a schematic diagram illustrating a capsule endoscope system according to an embodiment of the disclosure.
Fig. 2 is a structure diagram illustrating an external appearance of a capsule endoscope according to an embodiment of the disclosure.
Fig. 3 is a schematic diagram illustrating an internal structure of a capsule endoscope according to an embodiment of the disclosure.
Fig. 4 is a flow diagram illustrating a method for controlling movement of a capsule endoscope in a tissue cavity according to an embodiment of the disclosure.
Fig. 5 is another flow diagram illustrating a method for controlling movement of a capsule endoscope in a tissue cavity according to an embodiment of the disclosure.
Fig. 6 is a schematic diagram illustrating an exemplary path of movement of a capsule endoscope in a stomach according to an embodiment of the disclosure.
Fig. 7 is a schematic diagram illustrating another moving path for movement of a capsule endoscope in a tissue cavity according to an embodiment of the disclosure.
Fig. 8 is a schematic view illustrating shooting of a capsule endoscope floating on a liquid surface in a tissue cavity according to an embodiment of the disclosure.
Fig. 9 is another schematic view illustrating shooting of a capsule endoscope floating on a liquid surface in a tissue cavity according to an embodiment of the disclosure.
Fig. 10 is a schematic diagram illustrating a magnetic control device according to an embodiment of the disclosure.
Fig. 11 is another schematic diagram illustrating a magnetic control device according to an embodiment of the disclosure.

### Reference numerals

1, capsule endoscope system; 2, subject; 3, tissue cavity; 4, liquid; 10, capsule endoscope; 11, first magnet; 12, imaging device; 13, transmitting antenna; 14, circuit assembly; 15, power supply; 20, examination couch; 30, magnetic control device; 31, second magnet; 32, first induction coil; 33, second induction coil; 34, third induction coil; 40, operating device; 50, communication device; 60, storage unit; 70, display device.

### DETAILED DESCRIPTION

Hereinafter, preferred embodiments of the disclosure will be described in detail with reference to the accompanying drawings. In the following description, the same reference numerals are given to the same parts, and repeated descriptions are omitted. In addition, the drawings are only schematic and the scale of the dimensions of the parts relative to each other or the shape of the parts etc. may differ from practical conditions.

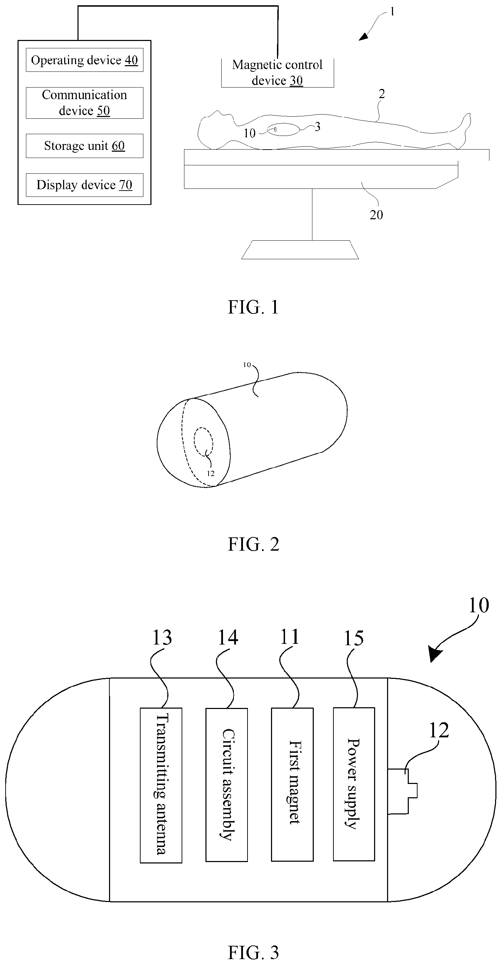

Fig. 1 is a schematic diagram illustrating a capsule endoscope system 1 according to an embodiment of the disclosure. Fig. 2 is a structure diagram illustrating an external appearance of capsule endoscope 10 according to an embodiment of the disclosure. Fig. 3 is a schematic diagram illustrating an internal structure of capsule endoscope 10 according to an embodiment of the disclosure.

Capsule endoscope system 1 according to the present embodiment may include capsule endoscope 10 located in tissue cavity 3 of subject 2, examination couch 20 bearing subject2, magnetic control device 30 for magnetically controlling capsule endoscope 10, and operating device 40 for operating examination couch 20 and magnetic control device 30 (see Fig. 1). In addition, capsule endoscope system 1 may further include communication device 50 that wirelessly communicates with capsule endoscope 10 within subject 2, storage unit 60 that stores various information such as an in-vivo image of subject 2, and display device 70 that displays various information such as an in-vivo image of subject 2 shot by capsule endoscope 10 (see Fig. 1).

Capsule endoscope 10 according to the present embodiment is a medical device configured to be capable of being introduced into tissue cavity 3 of subject 2 and shaped as a capsule. Apparently, capsule endoscope 10 may be a capsule-type shell (see Fig. 2). For the capsule-type shell of capsule endoscope 10, it may be a capsule-type shell formed in a size that can be introduced into the interior of subject 2. Openings of both ends of the capsule-type shell are plugged by a dome-shaped shell in a dome shape to maintain a liquid-tight state. The dome-shaped shell is a transparent optical dome that transmits light in a specified wavelength band(e.g., visible light). In another aspect, the cylindrical shell is a substantially opaque shell.

In this embodiment, tissue cavity 3 may be a digestive cavity such as a stomach, an esophagus, a large intestine, a colon, a small intestine, etc. In addition, in some instances, tissue cavity 3 may also be a non-digestive cavity such as an abdominal cavity, a thoracic cavity, etc. For a digestive cavity such as a stomach, an esophagus, a large intestine, etc., capsule endoscope 10 may enter the digestive cavity by consumption, while for a non-digestive cavity, capsule endoscope 10 may be placed into the non-digestive cavity by opening a minimally invasive opening by a clinical surgery.

In the present embodiment, capsule endoscope 10 includes at least magnet 11 (first magnet 11) and imaging device 12 for shooting in tissue cavity (e.g., stomach) 3. Capsule endoscope 10 shoots in-vivo images of subject 2 through imaging device 12. In addition, transmitting antenna 13 for wireless transmission with communication device 50, circuit assembly 14, power supply 15, etc. are arranged inside capsule endoscope 10 (see Fig. 3).

In some examples, capsule endoscope 10 may have imaging device 12 on one side. In other examples, capsule endoscope 10 may have two imaging devices 12 on both sides, where capsule endoscope 10 is capable of capturing images of both sides simultaneously.

In addition, as described above, an assembly (circuit assembly 14) adapted to imaging device 12 is also provided within capsule endoscope 10. In some examples, circuit assembly 14 may include an illumination portion such as an LED or the like, an optical system such as a condenser lens or the like, and an image pickup element implemented using a CCD or CMOS or the like.

In some examples, circuit assembly 14 may include control circuitry to control various components within capsule endoscope 10. Power supply 15 may be implemented using a switching circuit and a button-type battery or the like. In some examples, the above-described capsule endoscope 10 may be supplied with power based on the control of the control circuit when switched to the ON state by the switching circuit.

In some examples, by magnetic control device 30 disposed outside subject 2, capsule endoscope 10 can be moved in tissue cavity 3 under the action of an external magnetic field, while the inner wall (e.g., the stomach wall) of tissue cavity 3 can be shot, and image signals of the acquired in-vivo image are wirelessly transmitted to communication device 50 outside subject 2 and displayed on display device 70.

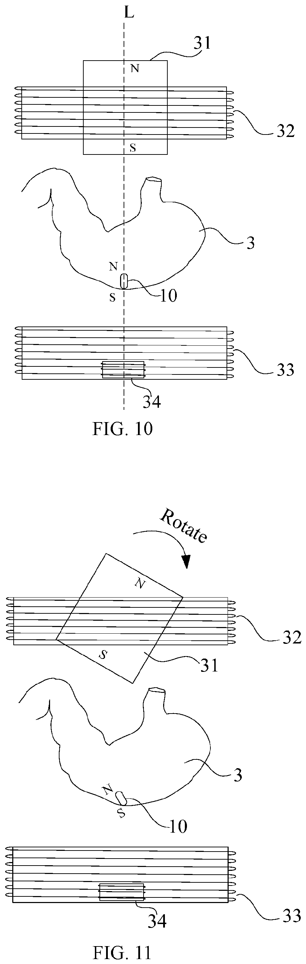

In this embodiment, magnetic control device 30 may be composed of a plurality of coils and magnets. In some examples, magnetic control device 30 may include second magnet 31 and first induction coil 32 arranged around second magnet 31 (see Fig. 10). Magnetic control device 30 may generate a three-dimensional external magnetic field such as a rotating magnetic field or a gradient magnetic field using electric power supplied from an electric power supply device. In particular, magnetic control device 30 is capable of generating at least a variable magnetic field with a gradient in the vertical direction. Magnetic control device 30 applies an external variable magnetic field for capsule endoscope 10 inside subject 2 placed on examination couch 20, and generates a magnetic attraction force to first magnet 11 inside subject 2 by the action of the external variable magnetic field, thereby guiding capsule endoscope 10 to a desired position inside tissue cavity 3. In some examples, examination couch 20 may be placed on the ground or on a horizontal plane, where subject 2 may lie flat on examination couch 20 for cavity wall examination of tissue cavity 3.

In this embodiment, operating device 40 can move examination couch 20 and subject 2 on examination couch 20 relative to magnetic control device 30, for example, in an XYZ three-dimensional coordinate position, thereby moving examination couch 20 and subject 2 on examination couch 20 to an appropriate position. Operating device 40 can apply an external variable magnetic field generated by magnetic control device 30 to capsule endoscope 10 inside subject 2, thereby changing the movement of capsule endoscope 10. In this case, capsule endoscope 10 inside subject 2 can be positioned in a three-dimensional space formed with the external magnetic field generated by magnetic control device 30.

In addition, operating device 40 may be implemented by using an input device such as a keyboard, a mouse, a joystick, etc. to input various information to operating device 40 according to an input operation of a user such as a doctor or a nurse.

In this embodiment, communication device 50 may be connected with a receiving antenna (not shown) disposed outside subject 2 (e.g., the body surface), and wirelessly communicate with transmitting antenna 13 of capsule endoscope 10 inside subject 2. In some examples, communication device 50 may receive wireless signals and position information from capsule endoscope 10 via the receiving antenna, perform demodulation processing, and extract image signals and position information contained in the wireless signals, etc.

In addition, in some examples, display device 70 may demodulate a wireless signal from capsule endoscope 10 acquired from communication device 50 and display image information corresponding to the image signal, i.e., an in-vivo image of subject 2. Therein, a group of in-vivo images of subject 2 generated by display device 70 may be stored in storage unit 60.

In the present embodiment, magnetic control device 30 may generate a variable magnetic field to guide and control the moving direction of capsule endoscope 10. Operating device 40 is controlled by an operator such as a doctor or the like, so that capsule endoscope 10 positioned in subject 2 is moved to an appropriate position by magnetic control device 30, and an image inside tissue cavity 3 is captured by capsule endoscope 10.

As described above, capsule endoscope 10 is a medical device formed so as to be capable of being introduced into tissue cavity 3 of subject 2 and shaped like a capsule, and includes at least magnet 11 (first magnet 11) and imaging device 12 for taking images in tissue cavity (e.g., the stomach) 3. Capsule endoscope 10 shoots an in-vivo image of subject 2 through imaging device 12. Magnetic control device 30 may be composed of a plurality of coils and magnets, and magnetic control device 30 may generate a three-dimensional external magnetic field such as a rotating magnetic field or a gradient magnetic field using electric power supplied from an electric power supply device. Magnetic control device 30 applies an external variable magnetic field for capsule endoscope 10 inside subject 2 placed on examination couch 20, and generates a magnetic attraction force to first magnet 11 inside subject 2 by the action of the external variable magnetic field, thereby guiding capsule endoscope 10 to a desired position inside tissue cavity 3.

As an important component of capsule endoscope system 1, magnetic control device 30 mainly controls capsule endoscope 10 to move in tissue cavity 3 by applying a variable magnetic field for capsule endoscope 10 so as to acquire images inside tissue cavity 3.

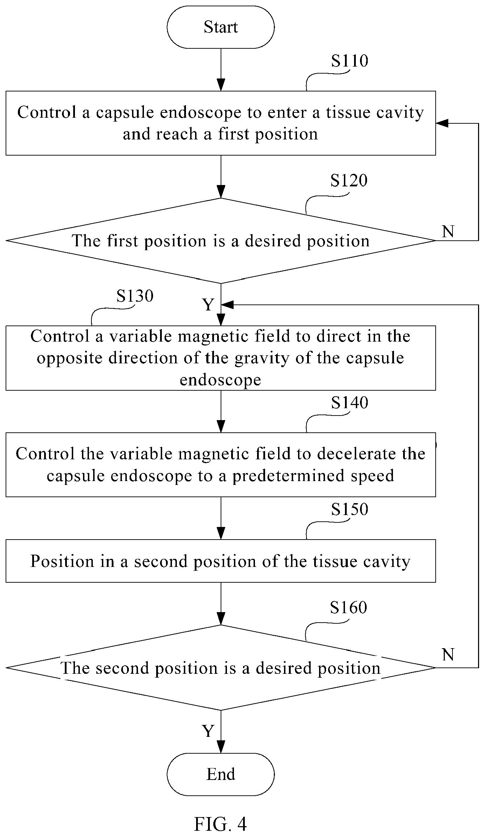

Hereinafter, with reference to Figs. 4 to 6, a method for controlling the movement of capsule endoscope 10 in tissue cavity 3 by magnetic control device 30 according to the disclosure will be described in detail. In addition, for ease of illustration, the disclosure is primarily directed to the method for controlling the movement of capsule endoscope 10 in tissue cavity 3, taking the stomach as an example. However, those skilled in the art will readily understand that the method for controlling the movement of capsule endoscope 10 in tissue cavity 3 of the disclosure may be applicable to other tissue cavities 3, such as a digestive cavity (an esophagus, a large intestine, a colon, a small intestine, etc.), or may be adapted slightly without involving any inventive effort.

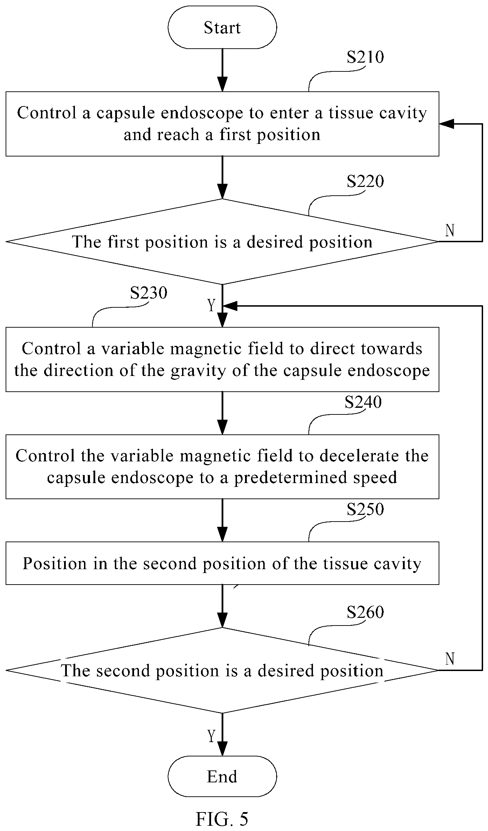

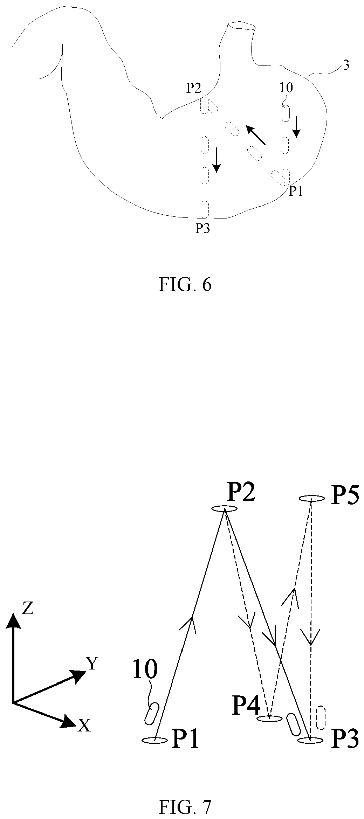

Fig. 4 is a flow diagram illustrating a method for controlling movement of capsule endoscope 10 in tissue cavity 3 according to an embodiment of the disclosure. Fig. 5 is another flow diagram illustrating a method for controlling movement of capsule endoscope 10 in tissue cavity 3 according to an embodiment of the disclosure. Fig. 6 is a schematic diagram illustrating an exemplary path of movement of capsule endoscope 10 in a stomach according to an embodiment of the disclosure.

At the start of the examination of the stomach of subject 2, capsule endoscope 10 may be introduced into tissue cavity 3 of the subject through a natural channel of the human body (e.g., mouth, etc.) or through a small surgical incision. At this time, subject 2 can be laid on examination couch 20 on one's back.

In some examples, capsule endoscope 10 may be arranged not to be viewed (image acquisition) until a prescribed time has elapsed or until a prescribed location has been reached; and after the prescribed time has elapsed or upon confirmation that capsule endoscope 10 has arrived in tissue cavity 3 to be examined by observing an image or the like, power supply 15 in capsule endoscope 10 is activated to start the operation of capsule endoscope 10.

Next, a variable magnetic field may be generated to capsule endoscope 10 by magnetic control device 30 disposed outside subject 2, and a driving force may be generated to capsule endoscope 10 by controlling the variable magnetic field, thereby moving capsule endoscope 10 from first position Pl in tissue cavity 3 to second position P2 towards the opposite side in tissue cavity 3.

It should be noted that neither first position Pl nor second position P2 is particularly limited here, as long as it is located in tissue cavity 3. For example, as shown in Fig. 6, both first position Pl and second position P2 may be located on the cavity wall (here, the stomach wall) in the tissue cavity 3. In some examples, first position Pl may be located on the cavity wall in tissue cavity 3, and second position P2 may be located in a place in tissue cavity 3 that does not contact the cavity wall. In other examples, both first position Pl and second position P2 may be located in places in tissue cavity 3 which do not contact the chamber wall.

In some cases, after capsule endoscope 10 enters tissue cavity 3 of subject 2, capsule endoscope 10 reaches the bottom (for example, first position PI) of tissue cavity 3 (for example, the stomach). At this time, capsule endoscope 10 is controlled by controlling the variable magnetic field so as to adjust the lens orientation of capsule endoscope 10, so that capsule endoscope 10 is firstly located at the bottom of tissue cavity 3 to shoot, and acquire an in-vivo image of the inner wall of tissue cavity 3 (step S 110). Additionally, in some examples, it may be determined whether first position P1 is the desired position (step S120). If first position Pl is not the desired position, return to step S110 to redirect capsule endoscope 10 with the variable magnetic field and position capsule endoscope 10 by a method such as magnetic field positioning. If first position Pl is the desired position, in order to move capsule endoscope 10 to the opposite side of tissue cavity 3 (i.e., move capsule endoscope 10 in the opposite direction of its gravity), the variable magnetic field can be controlled so that capsule endoscope 10 generates an upward component force by the actions of the magnetic force of the variable magnetic field, the gravity of capsule endoscope 10 itself, and the supporting force and/or frictional force at the bottom of tissue cavity 3, and capsule endoscope 10 is moved to the opposite side of tissue cavity 3 (step S130). Here, the desired position may be, for example, a position at which a clear image can be acquired.

In addition, when capsule endoscope 10 approaches second position P2, the variable magnetic field may be controlled to decelerate capsule endoscope 10 to a predetermined speed (step S140). In other examples, it may also decelerate to less than the predetermined speed in step S140. Next, capsule endoscope 10 is known to be in second position P2 by a method such as magnetic field positioning, and capsule endoscope 10 is enabled to take pictures of the cavity wall (e.g., stomach wall) of tissue cavity 3 at second position P2 (step S150). Thus, it is possible to prevent that the impact force generated in the accelerated moving state of capsule endoscope 10damages the tissue on the side wall of the tissue cavity 3. In some examples, it may be determined whether the second position is a desired position (step S160). If second position P2 is not the desired position, it returns to step S130, capsule endoscope 10 may be redirected with the variable magnetic field, and capsule endoscope 10 is positioned by a method such as magnetic field positioning. Here, second position P2 is not particularly limited and may be any point within tissue cavity 3 or may be a side wall on the opposite side with respect to the bottom of tissue cavity 3.

In other cases, first position Pl may be any position within tissue cavity 3 other than the bottom. At this time, capsule endoscope 10 is controlled by controlling the variable magnetic field and reaches the first position (step S210). In some examples, it may be determined whether the first position is a desired position (step S220). If first position Pl is not the desired position, it returns to step S210, capsule endoscope 10 is redirected with the variable magnetic field, and capsule endoscope 10 is positioned with a method such as magnetic field positioning. If first position Pl is the desired position, in order to move capsule endoscope 10 to the opposite side of tissue cavity 3 (i.e., move capsule endoscope 10 in the direction of gravity thereof), the variable magnetic field can be controlled so that capsule endoscope 10 generates an upward component force by the actions of the magnetic force of the variable magnetic field and the gravity of capsule endoscope 10 itself, and capsule endoscope 10 is moved to the opposite side of tissue cavity 3 (step S230).

In addition, when capsule endoscope 10 approaches second position P2, the variable magnetic field is controlled to decelerate capsule endoscope 10 to a predetermined speed (step S240). In other examples, it may also decelerate to less than this predetermined speed in step S140. Next, capsule endoscope 10 is known to be in second position P2 with a method such as magnetic field positioning, and capsule endoscope 10 is enabled to shoot the cavity wall (e.g., stomach wall) of tissue cavity 3 at second position P2 (step S250). Thus, it is possible to prevent that the impact force generated in the accelerated moving state of capsule endoscope 10 damages the tissue on the side wall of tissue cavity 3. In some examples, it may be determined whether the second position is a desired position (step S260). If second position P2 is not the desired position, it returns to step S230, capsule endoscope 10 may be redirected with the variable magnetic field, and capsule endoscope 10 is positioned with a method such as magnetic field positioning.

In some examples, the above-described steps S110 to S160 and steps S210 to S260 shown in Figs. 5 and 6 are repeated until the image shoot within tissue cavity 3 is completed.

In this embodiment, magnetic control device 30 is configured to generate a variable magnetic field for capsule endoscope 10, including a base magnetic field generated by second magnet 31 and an induced magnetic field generated by first induction coil 32. The direction of magnetic axis L of the induction coil is maintained in a fixed orientation, so that capsule endoscope 10 is always located in magnetic axis L of first induction coil 32 under the traction of the variable magnetic field (refer to Fig. 10).

In some examples, the magnitude of the magnetic force of the variable magnetic field may be adjusted by changing the relative position of a pole of second magnet 31 with respect to first magnet 11 (i.e., magnet 11 in capsule endoscope 10). In some examples, the movement and deviation of capsule endoscope 10 may be controlled by a variable magnetic field produced from, for example, deviating second magnet 31 to deviate the polarities of N and S poles of second magnet 31, or moving second magnet 31 to change the relative position of the poles of second magnet 31 with respect to first magnet 11. In other embodiments, the movement and deviation of capsule endoscope 10 may be controlled by a variable magnetic field produced by moving the position of subject 2 such that the relative position of the pole of second magnet 31 with respect to first magnet 11 is varied.

It will be understood that first magnet 11 of capsule endoscope 10 of the present embodiment may be fixed to capsule endoscope 10, so that the deviation of the polarity of first magnet 11 may cause the deviation of the lens of capsule endoscope 10.

Further, in some examples, the magnitude of the variable magnetic field may be controlled by varying the magnitude of the current of first induction coil 32, while the direction of the variable magnetic field may be controlled by varying the relative position of first induction coil 32 with respect to first magnet 11. Additionally, in some examples, the change of the relative position of first induction coil 32 relative to first magnet 11 may be accomplished by moving first induction coil 32. In other examples, the change in the relative position of first induction coil 32 with respect to first magnet 11 may also be accomplished by moving the position of subject 2.

Further, in some examples, the magnitude of the magnetic force of the variable magnetic field may be changed by changing the direction of the current of first induction coil 32 to change the polarity of the magnetic field generated by first induction coil 32.

It will be understood that the path optimization of capsule endoscope 10 in the space of tissue cavity 3 can be achieved by means of the base magnetic field generated by second magnet 31 and the induced magnetic field generated by first induction coil 32, and by means of either of or a resultant force of both of the two magnetic forces, while the moving speed of capsule endoscope 10 can be controlled mainly by controlling the intensity of the current of first induction coil 32. Thereby, it not only can realize the comprehensive inspection of tissue cavity 3, but also can protect the cavity wall of tissue cavity 3.

In this embodiment, in some examples, first position Pl may be a cavity bottom position of tissue cavity 3. Magnetic control device 30 is configured to control the variable magnetic field to generate an upward magnetic force when capsule endoscope 10 is positioned in first position P1 during the movement of capsule endoscope 10 from first position P1 to second position P2. At the same time, capsule endoscope 10 generates an upward component force under the supporting force and/or friction force of the bottom of tissue cavity 3 and the downward gravity force of capsule endoscope 10, so that capsule endoscope 10 is accelerated. When capsule endoscope 10 approaches second position P2, capsule endoscope 10 loses the supporting force and/or the frictional force of the bottom of the stomach at this time, and capsule endoscope 10 can be decelerated to a predetermined speed, or less, by controlling the variable magnetic field to balance the gravity of capsule endoscope 10 itself.

In some examples, second position P2 may be any position in the space of tissue cavity 3 with respect to first position Pl, where tissue cavity 3 can be detached from the obstruction of the mucous membrane or protrusion structure or the like at the bottom of tissue cavity 3, and capsule endoscope 10 is decelerated to the predetermined speed upon reaching the position, thereby shooting tissue cavity 3. In other implementation examples, second position P2 may be another side wall of tissue cavity 3 relative to first position P1. In this case, since capsule endoscope 10 is close to the side wall of tissue cavity 3, which corresponds to giving a fulcrum to capsule endoscope 10, thus capsule endoscope 10 can stably shoot at this fulcrum based on the control of the variable magnetic field.

In some examples, magnetic control device 30 is configured to control a variable magnetic field to accelerate capsule endoscope 10 to an initial speed when capsule endoscope 10 is in first position P1, and move it for a predetermined distance while the initial speed is maintained, then the variable magnetic field is controlled to decelerate capsule endoscope 10 to a predetermined speed when capsule endoscope 10 approaches second position P2, during the movement of capsule endoscope 10 from first position P1 to second position P2. In this way, capsule endoscope 10 undergoes uniform motion in the middle of the moving process, so that the buffering speed when capsule endoscope 10 decelerates can be reasonably controlled; and on the other hand, the definition of images shot by capsule endoscope 10 at the uniform motion stage can be facilitated.

In this embodiment, when capsule endoscope 10 is located in second position P2, the side of capsule endoscope 10 facing first position Pl captures tissue cavity 3. In this case, tissue cavity 3 can be shot using the deviation of the lens of imaging device 12 by controlling the deviation of second magnet 31 (see Fig. 10 described later) to control the orientation of imaging device 12. Additionally, in some examples, second position P2 may be any position in the space of tissue cavity 3 opposite to first position P1. In other embodiments, second position P2 may be a sidewall of tissue cavity 3 opposite to first position P1.

In addition, in the present embodiment, first position Pl may preferably be a sidewall on one side of tissue cavity 3, and second position P2 may preferably be a sidewall on the other side of tissue cavity 3. Thereby, capsule endoscope 10 can be stabilized by means of the supporting force of the side wall, so that capsule endoscope 10 can shoot more stably under the control of the variable magnetic field.

Referring again to Fig. 6, in the present embodiment, the process of moving capsule endoscope 10 from first position P1 of the stomach to second position P2 of the stomach and back again to third position P3 of the stomach is a shuttle process. First position P1 and third position P3 can be located on one side of the stomach, and second position P2 can be located on the other side of the stomach; and during the shuttle process, the variable magnetic field can be controlled to deviate capsule endoscope 10 so as to deviate third position P3 of capsule endoscope 10 from first position P1.

In some examples, the shuttle may be a shuttle in the stomach space so that damage to stomach sidewall tissue due to impact forces generated by capsule endoscope 10 can be avoided. In other embodiments, the shuttle may be a shuttle between the side walls of the stomach, so that a more complete examination on the stomach may be achieved, avoiding the presence of missed detections to concealed locations of the stomach.

In this embodiment, magnetic control device 30 is configured to move tissue cavity 3 in a direction forming an included angle with the direction of magnetic axis L so that the position of first magnet 11 deviates from magnetic axis L, and second magnet 31 generates a magnetic force acting on capsule endoscope 10 in the direction of magnetic axis L, when capsule endoscope 10 is located at first position Pl or second position P2 of tissue cavity 3. Thereby, capsule endoscope 10 can be driven to move to a specific path conveniently by the cooperation of the base magnetic field and the induced magnetic field. In this case, by moving the position of subject 2, capsule endoscope 10 can be more stably controlled.

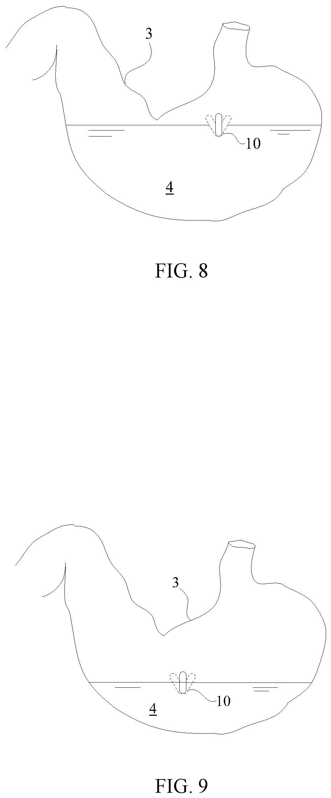

Fig. 8 is a schematic view illustrating shooting of capsule endoscope 10 floating on a liquid surface in tissue cavity 3 according to an embodiment of the disclosure. Fig. 9 is another schematic view illustrating shooting of capsule endoscope 10 floating on a liquid surface in tissue cavity 3 according to an embodiment of the disclosure.

In some examples, tissue cavity 3 may be filled with liquid 4 (see Fig. 8 or Fig. 9). In this case, the specific gravity value of capsule endoscope 10 may be set to be less than and close to the specific gravity value of liquid 4 supplied to tissue cavity 3. In some examples, for example, when liquid 4 is water, the specific gravity value of capsule endoscope 10 may be set to be less than 1 and close to 1. In addition, the center of gravity of capsule endoscope 10 can be set to deviate to one side of the capsule-type shell. In this case, capsule endoscope 10 can more stably float on the liquid surface under the guidance of the center of gravity in liquid 4. In this case, it is convenient to take images in tissue cavity 3 using capsule endoscope 10, and the problem of instability of the taken image due to the fluctuation influence of the liquid surface is reduced. In some examples, liquid 4 may be water, soda, milk, or other various liquids harmless to the human body.

In addition, liquid 4 can form a liquid surface in tissue cavity 3. In this case, tissue cavity 3 is shot by controlling the variable magnetic field of magnetic control device 30 so as to make capsule endoscope 10 on the liquid surface, maintain a relative position with respect to tissue cavity 3, and move subject 2 (i.e., move tissue cavity 3). In addition, since capsule endoscope 10 is positioned on the liquid surface, capsule endoscope 10 can float on the position of the liquid surface by controlling magnetic control device 30 in a state that the liquid surface is relatively stable; and tissue cavity 3 can be shot by adjusting the orientation of imaging device 12, and the position of tissue cavity 3 (i.e. subject 2) can also be moved, so that capsule endoscope 10 can move in all directions on the level of liquid 4.

Referring to Fig. 9, capsule endoscope 10 can be moved longitudinally and perpendicularly to the horizontal plane of liquid 4 by using the change of the position of liquid 4. In some examples, the liquid surface may be gradually lowered using the absorption function of tissue cavity 3 itself. In other examples, it is also possible to gradually raise the position of the liquid surface of liquid 4 by gradually filling tissue cavity 3 with water.

In the case that the liquid surface of liquid 4 changes, capsule endoscope 10 can shoot tissue cavity 3 along with the gradual change of liquid 4 while floating on liquid 4, so that the area to be shot in tissue cavity 3 can be basically covered. In addition, in such a shooting process, it is also possible to perform rotational shooting of capsule endoscope 10, so that the shooting coverage rate of tissue cavity 3 can be further improved.

It will be understood that capsule endoscope 10 can stably shoot on the position of the liquid surface by utilizing the buoyancy of liquid 4, the gravity of capsule endoscope 10 and the magnetic force of the variable magnetic field, and tissue cavity 3 can be shot more conveniently by adjusting the position of the liquid surface and moving capsule endoscope 10 on the liquid surface. In addition, in this way, capsule endoscope 10 can stably rotate and shoot at any spatial position in tissue cavity 3, so that images can be taken omni-directionally in the body.

In some examples, the level of liquid 4 formed in tissue cavity 3 may be no less than 50 mm, which ensures that capsule endoscope 10 floats on the liquid surface. In addition, it is possible to float capsule endoscope 10 in a specific position within tissue cavity 3 by changing the magnitude and direction of the magnetic force of the variable magnetic field, while the wall of tissue cavity 3 is shot by changing the orientation of imaging device 12. In addition, in some examples, it is also possible to adjust the position where capsule endoscope 10 floats in liquid 4 by changing the magnitude of the magnetic force of the variable magnetic field, thereby making it more advantageous for capsule endoscope 10 to take pictures in different positions.

In some examples, tissue cavity 3 may be moved relative to capsule endoscope 10 to shoot tissue cavity 3, i.e., tissue cavity 3 is moved relative to capsule endoscope 10 by maintaining the position of capsule endoscope 10 with magnetic control device 30. In other examples, the position of capsule endoscope 10 may be changed to shoot tissue cavity 3 by changing the position of magnetic control device 30.

In some examples, capsule endoscope 10 may also be suspended anywhere below the liquid surface for shooting by changing the specific gravity value of capsule endoscope 10 and the variable magnetic field.

Fig. 10 is a schematic diagram illustrating a magnetic control device 30 according to an embodiment of the disclosure. Fig. 11 is another schematic diagram illustrating magnetic control device 30 according to an embodiment of the disclosure.

In this embodiment, magnetic control device 30 may include a magnet (second magnet) 31 and first induction coil 32. Therein, first induction coil 32 is arranged on the same side as second magnet 31. In this way, the magnetic field force generated by first induction coil 32 and the magnetic field force generated by second magnet 31 can be more concentrated, and capsule endoscope 10 can be more easily controlled.

Additionally, in some examples, magnetic control device 30 may also include second induction coil 33 arranged on a different side from first induction coil 32. Therein, first induction coil 32 is arranged on the same side as second magnet 31, and second induction coil 33 is arranged on a different side from second magnet 31. By arranging first induction coil 32 and second induction coil 33 on different sides of subject 2, the magnitudes of the magnetic forces in the up-down direction can be balanced, thereby enabling capsule endoscope 10 to move more stably within tissue cavity 3.

In addition, in the present embodiment, magnetic control device 30 may further include third induction coil 34. In some examples, third induction coil 34 may generate a magnetic force acting on capsule endoscope 10 to position capsule endoscope 10 at a predetermined position within tissue cavity 3. That is, third induction coil 34 may has a function of positioning capsule endoscope 10.

In this embodiment, the diameter of third induction coil 34 may be smaller than the diameter of first induction coil 32 and the diameter of second induction coil 33. For example, the diameter of third induction coil 34 is generally set to 1-15 cm, and the diameters of first induction coil 32 and second induction coil 33 may be set to 20-45 cm. In this case, for capsule endoscope 10 having a small volume, when capsule endoscope 10 is within the range of first induction coil 32 and second induction coil 33, a more accurate positioning of capsule endoscope 10 within tissue cavity 3 can be achieved using third induction coil 34.

Further, in the present embodiment, third induction coil 34 may be provided on the same side as second induction coil 33. For example, third induction coil 34 and second induction coil 33 may be provided on the side close to the ground. In this way, the magnetic field forces generated by first induction coil 32 and second magnet 31 located on the opposite side of second induction coil 33 can be more easily balanced by the gravity of capsule endoscope 10 itself.

In addition, in the present embodiment, imaging device 12 is provided at both ends of capsule endoscope 10, respectively. In this way, the operation such as rotation of capsule endoscope 10 can be reduced, whereby tissue cavity 3 can be shot more easily and omni-directionally.

In addition, in the present embodiment, the center of gravity of capsule endoscope 10 may be closer to the other end with respect to one end. By means of the design, the position of the center of gravity of capsule endoscope 10 can be stabilized, and capsule endoscope 10 can shoot more stably on the cavity wall of tissue cavity 3 or by floating on the liquid surface.

In the present embodiment, when capsule endoscope 10 is positioned on the side wall of tissue cavity 3, the orientation of imaging device 12 at the other end of capsule endoscope 10 is adjusted and shoot, with one end of capsule endoscope 10 located on the side wall of tissue cavity 3 as a fulcrum by controlling the variable magnetic field of magnetic control device 30. In this case, capsule endoscope 10 can stably shoot towards the other side of tissue cavity 3 by means of the supporting force of the side wall of tissue cavity 3 and by adjusting the orientation of imaging device 12.

In some examples, referring to Fig. 11, magnetic control device 30 is configured to control the variable magnetic field by changing the position of the pole of second magnet 31 relative to first magnet 11. In this case, the control of the deviation and movement of capsule endoscope 10 is achieved by deviating the polarity of second magnet 31 and simultaneously changing the relative position of second magnet 31 relative to first magnet 11.

In this embodiment, imaging device 12 of capsule endoscope 10 can perform automatic shooting at specified time intervals within tissue cavity 3. For example, it shoots every 0.1 seconds, every 0.2 seconds, every 0.3 seconds, or every 0.5 seconds, etc. Thus, it is possible to activate capsule endoscope 10 before or after capsule endoscope 10 enters tissue cavity 3 so that capsule endoscope 10 automatically shoots in tissue cavity 3, to wirelessly transmit all the captured images to external communication device 50 via transmitting antenna 13, and finally to screen the images where the pathological change may occur through display device 70.

In some examples, imaging device 12 of capsule endoscope 10 may automatically take images in tissue cavity 3 at prescribed displacement intervals. For example, it is possible to take images at a predetermined displacement interval of about 1 mm to 11 mm. For another example, it is preferable to take images at a displacement interval of 2 mm to 8 mm, so that the images taken by imaging device 12 have an area overlap of 10% or more. In this way, the pictures taken by imaging device 12 can be continuously generated, thereby helping to identify the location of tissue cavity 3.

In some examples, as shown in Fig. 10, when second magnet 31 is in the initial position, the direction of magnetic axis L may be a vertical direction, and magnetic axis L may pass through second magnet 31. Thereby, the axis of second magnet 31 in the vertical direction and magnetic axis L of first induction coil 32 can be made to coincide, so that capsule endoscope 10 can be maintained substantially on magnetic axis L during the control of the movement of capsule endoscope 10.

In the present embodiment, in some examples, first induction coil 32 may be arranged around second magnet 31, and the diameter of first induction coil 32 may be larger than that of a sphere as second magnet 31. Thus, the geometric center of the magnet 31 and the geometric center of first induction coil 32 can be made to coincide, so that the generated magnetic field force can be relatively concentrated, and in addition, the space occupied by magnetic control device 30 can be saved.

In the present embodiment, as described above, second magnet 31 may be a sphere. In this case, the deviation of the polarity of first magnet 11 in capsule endoscope 10 can be driven by changing the deviation of the polarity of the sphere, so that the deviation of the shooting angle of capsule endoscope 10 can be driven. In other examples, second magnet 31 may also be an ellipsoid or a cylinder or the like. For example, when second magnet 31 is a cylinder, second magnet 31 is arranged around first induction coil 32 in such a manner that second magnet 31 is rotatable around a point as the center at which it intersects with the magnetic axis of first induction coil 32. Specifically, when second magnet 31 is a cylinder, the diameter of first induction coil 32 can be made larger than the larger dimension of the height of the cylinder, the diameter of the bottom surface of the cylinder, or the diagonal of the rectangle of the front view of the cylinder, so that the cylinder can freely rotate in the space surrounded by first induction coil 32.

In the present embodiment, the magnetic fields of first magnet 11, second magnet 31, first induction coil 32, second induction coil 33, etc. can be detected respectively by using a magnetic sensor array (not shown), and the position of the magnetic sensor array with respect to capsule endoscope 10 can be calculated based on the model of the magnetic dipole of capsule endoscope 10 so as to obtain the positioning place of capsule endoscope 10 with respect to tissue cavity 3.

In particular, the magnetic sensor array may be at least one three-axis magnetic sensor, two or more two-axis magnetic sensors, or three or more one-axis magnetic sensors. In some examples, the magnetic sensor array may be disposed on a support (not shown) that is relatively stationary with respect to first induction coil 32 and second induction coil 33 and remains relatively stationary with respect to the ground. In some examples, the support is disposed on couch 20, and subject 2 may be positioned on examination couch 20 and moved as couch 20 moves while an examination is performed.

The background magnetic field (Xₘ₀, Yₘ₀, Zₘ₀) generated by magnetic control device 30 consisting of first induction coil 32 and second magnet 31 can be measured in advance before capsule endoscope 10 enters tissue cavity 3. When capsule endoscope 10 is positioned anywhere in tissue cavity 3, the intensity of the magnetic sensor received by the magnetic sensor is set to be (Xs, Ys, Zs), and the magnetic field generated by magnetic control device 30 is (Xₘ₁, Yₘ₂, Zₘ₃). In this case, since the relative positions of first induction coil 32 and second magnet 31 remain fixed, it is possible to adjust (Xₘ₁, Ym₂, Zₘ₃) to (Xₘ₀, Yₘ₀, Zₘ₀) by adjusting the current magnitude of first induction coil 32 or the deviation direction of second magnet 31. In this case, the magnetic field intensity generated by first magnet 11 in capsule endoscope 10 (Xc, Yc, Zc) = (X_{S}-Xₘ₀, Y_{S}-Yₘ₀, Z_{S}-Zₘ₀), and the relative position of capsule endoscope 10 with respect to the magnetic sensor array can be calculated by using the magnetic dipole model of first magnet 11 in capsule endoscope 10.

In addition, based on the positioning place acquired by the magnetic sensor array, the moving path of capsule endoscope 10 in tissue cavity 3 can be planned and adjusted, so that the shooting region of capsule endoscope 10 substantially covers tissue cavity 3. Thus, the moving path of the next step of capsule endoscope 10 can be reasonably optimized. For example, the concentration of the moving path of capsule endoscope 10 can be increased in a region where a pathological change may exist, so that capsule endoscope 10 can realize omni-directional and detailed shooting of tissue cavity 3.

In this embodiment, as described above, tissue cavity 3 may be a stomach. Since the stomach has a larger space compared with other tissue cavities 3, the magnetic control method disclosed in the disclosure can be fully utilized to plan the moving path of capsule endoscope 10 to achieve an omni-directional examination to the stomach.

As another example of path planning, Fig. 7 is a schematic diagram illustrating another moving path for movement of capsule endoscope 10 in tissue cavity 3 according to an embodiment of the disclosure. In Fig. 7, the structure of tissue cavity 3 is not shown for ease of illustration and. In addition, the XYZ coordinate system of the reference is shown beside for further illustration of the movement in three-dimensional space.

Referring to the above description, the process of moving capsule endoscope 10 from first position P1 of the stomach to second position P2 of the stomach and back again to third position P3 of the stomach is a shuttle process. Here, first position Pl and third position P3 may be located on one side of the stomach, and second position P2 may be located on the other side of the stomach. During the shuttle process, before capsule endoscope 10 is moved to second position P2 of the stomach, capsule endoscope 10 may also be moved to fourth position P4 other than first position P1 and second position P2, and fourth position P4 is not located in the plane formed by first position P1, second position P2 and third position P3. Thereby, all parts of tissue cavity 3 can be shot comprehensively.

In some examples, during the shuttle process, capsule endoscope 10 is also moved to fifth position P5 other than second position P2 and third position P3 before capsule endoscope 10 is moved to third position P3 of the stomach, wherein fifth position P5 is not located in the plane formed by first position P1, second position P2 and third position P3. Thereby, all parts of tissue cavity 3 can be shot comprehensively.

In other examples, first position Pl, second position P2, third position P3, fourth position P4, and fifth position P5 are all located on the inner wall of the stomach. Thereby, capsule endoscope 10 can shoot more stably with the aid of the supporting force of the inner wall of the stomach, and all parts of tissue cavity 3 can be shot comprehensively.

In addition, the above-described first position Pl and second position P2 on the opposite side are intended to illustrate that, under the magnetic control solution of the disclosure, capsule endoscope 10 can be moved to any spatial position of tissue cavity 3 and shoot, and capsule endoscope 10 can rotationally shoot in the process by deviating second magnet 31. Thus, its path of motion in tissue cavity 3 is in any three-dimensional direction, and is preferably an optimal path which covers tissue cavity 3.

In addition, in the above-described embodiment, the positioning of capsule endoscope 10 by using the magnetic sensor array is described, but it is also possible to replace the magnetic sensor array with an antenna array for positioning using an electric field measured by the antenna array.

Further, in the above-described embodiment, it is possible to arrange imaging device 12 dedicated to shooting and an illumination device dedicated to illumination, etc (not shown) in capsule endoscope 10. However, the disclosure is not limited thereto, and it is possible that only imaging device 12 is provided in capsule endoscope 10.

In addition, the position of the center of gravity of capsule endoscope 10 may be designed to be closer to the other side with respect to one side. In this case, it is convenient to control the form of capsule endoscope 10 in a vertical direction. However, the disclosure is not limited thereto, and the position of the center of gravity of capsule endoscope 10 may also be located in the geometric center of capsule endoscope 10.

Although the disclosure has been specifically described above with reference to the accompanying drawings and examples, it should be understood that the above description does not limit the disclosure in any way. Variations and modifications of the disclosure may occur to those skilled in the art as required without departing from the true spirit and scope of the disclosure, and such variations and modifications are intended to fall within the scope of the disclosure.

## Claims

1. A magnetic control device for a capsule endoscope, wherein the capsule endoscope comprises at least a first magnet and an imaging device and is located in a tissue cavity, **characterized in that**
the magnetic control device comprises a second magnet and a first induction coil arranged around the second magnet, and the magnetic control device is configured to generate a driving force to the capsule endoscope to move the capsule endoscope from a first position in the tissue cavity to a second position towards an opposite side in the tissue cavity; the capsule endoscope is decelerated to a predetermined speed when the capsule endoscope approaches the second position, the magnetic control device is configured to generate the variable magnetic field for the capsule endoscope, and the variable magnetic field comprises a base magnetic field generated by the second magnet and an induced magnetic field generated by the first induction coil; and the direction of a magnetic axis of the first induction coil is kept in a fixed orientation, and the variable magnetic field is generated by changing at least one of the relative position of a magnetic pole of the second magnet relative to the first magnet, the current magnitude of the first induction coil, and the current direction of the first induction coil.

2. The magnetic control device according to claim 1, **characterized in that**
the magnetic control device is configured to control the variable magnetic field to accelerate the capsule endoscope when the capsule endoscope is in the first position, and to control the variable magnetic field to decelerate the capsule endoscope to the predetermined speed when the capsule endoscope approaches the second position, during the movement of the capsule endoscope from the first position to the second position.

3. The magnetic control device according to claim 1, **characterized in that**
the magnetic control device is configured to control the variable magnetic field to accelerate the capsule endoscope to an initial speed and move a predetermined distance while maintaining the initial speed when the capsule endoscope is in the first position, then to control the variable magnetic field to decelerate the capsule endoscope to the predetermined speed when the capsule endoscope approaches the second position, during the movement of the capsule endoscope from the first position to the second position.

4. The magnetic control device according to claim 1, **characterized in that**
the first position is a side wall on one side of the tissue cavity, and the second position is a side wall on the other side of the tissue cavity.

5. The magnetic control device according to claim 1, **characterized in that**
the process of moving the capsule endoscope from the first position in the tissue cavity to the second position in the tissue cavity and back again to a third position in the tissue cavity is a shuttle process, wherein the first position and the third position are located on one side of the tissue cavity, and the second position is located on the other side of the tissue cavity;
the magnetic control device is configured to control the variable magnetic field to deviate the capsule endoscope, thereby deviating the third position of the capsule endoscope from the first position during the shuttle process.

6. The magnetic control device according to claim 5, **characterized in that**
the magnetic control device is configured to move the tissue cavity in a direction forming an included angle with the direction of the magnetic axis to deviate the position of the first magnet from the magnetic axis, and the second magnet generates a magnetic force acting on the capsule endoscope in the direction of the magnetic axis, when the capsule endoscope is in the first position or the second position in the tissue cavity.

7. A magnetic control device for a capsule endoscope, wherein the capsule endoscope comprises at least a first magnet and an imaging device and is located in a tissue cavity, **characterized in that**
the magnetic control device comprises a second magnet and a first induction coil arranged around the second magnet, the magnetic control device is configured to generate a variable magnetic field for the capsule endoscope, and the variable magnetic field comprises a base magnetic field generated by the second magnet and an induced magnetic field generated by the first induction coil; and the direction of the magnetic axis of the first induction coil is kept in a fixed orientation, and the variable magnetic field is generated by changing at least one of the relative position of a magnetic pole of the second magnet relative to the first magnet, the current magnitude of the first induction coil, and the current direction of the first induction coil.

8. The magnetic control device according to claim 1, **characterized in that**
the magnetic control device is configured so that the second magnet generates a first magnetic force to the capsule endoscope and the first induction coil generates a second magnetic force to the capsule endoscope during the movement of the capsule endoscope from the first position to the second position, wherein a deviating movement is generated to the capsule endoscope by forming an included angle between a magnetic force direction of the first magnetic force and a magnetic force direction of the second magnetic force.

9. The magnetic control device according to claim 1 or 8, **characterized in that** fluid is contained in the tissue cavity and a liquid surface is formed in the tissue cavity, and the magnetic control device is configured to control the variable magnetic field so that the capsule endoscope is on the liquid surface and remains in a position relative to the tissue cavity, and the tissue cavity is moved and shot inside.

10. The magnetic control device according to claim 1 or 8, **characterized in that** the first induction coil is arranged on the same side as the second magnet.

11. The magnetic control device according to claim 1 or 8, **characterized in that**
the magnetic control device further comprises a second induction coil arranged on a different side from the first induction coil, and the second induction coil is arranged on a different side from the second magnet.

12. The magnetic control device according to claim 1, **characterized in that**
the magnetic control device further comprises a third induction coil that generates a magnetic force acting on the capsule endoscope to position the capsule endoscope in a predetermined position within the tissue cavity.

13. The magnetic control device according to claim 12, **characterized in that**
the diameter of the third induction coil is smaller than the diameter of the first induction coil and the diameter of the second induction coil.

14. The magnetic control device according to claim 1 or 8, **characterized in that**
the magnetic control device is configured to adjust the orientation of the imaging device at the other end of the capsule endoscope with one end of the capsule endoscope located on the side wall in the tissue cavity being as a fulcrum by controlling the variable magnetic field, when the capsule endoscope is located on the side wall in the tissue cavity.

15. The magnetic control device according to claim 14, **characterized in that**
the magnetic control device is configured to control the variable magnetic field by changing a position of a magnetic pole of the second magnet relative to the first magnet.

16. The magnetic control device according to claim 1, **characterized in that**
the process of moving the capsule endoscope from the first position of the tissue cavity to the second position of the tissue cavity and back again to a third position of the tissue cavity is a shuttle process, wherein the first position and the third position are located on one side of the tissue cavity, and the second position is located on the other side of the tissue cavity;
during the shuttle process, the capsule endoscope is further moved to a fourth position other than the first position and the second position before the capsule endoscope is moved to the second position of the tissue cavity, wherein the fourth position is not located in a plane formed by the first position, the second position and the third position.

17. The magnetic control device according to claim 16, **characterized in that**
during the shuttle process, the capsule endoscope is further moved to a fifth position other than the second position and the third position before the capsule endoscope is moved to the third position of the tissue cavity, wherein the fifth position is not located in a plane formed by the first position, the second position and the third position.

18. The magnetic control device according to claim 17, **characterized in that**
the first position, the second position, the third position, the fourth position and the fifth position are all located on the inner wall of the tissue cavity.

19. A method for controlling movement of a capsule endoscope in a tissue cavity, wherein the capsule endoscope comprises at least a first magnet and an imaging device, **characterized in** comprising:
enabling the capsule endoscope to enter the tissue cavity;
generating a variable magnetic field for the capsule endoscope, and generating a driving force to the capsule endoscope by controlling the variable magnetic field, whereby moving the capsule endoscope from a first position in the tissue cavity to a second position towards an opposite side in the tissue cavity; and
controlling the variable magnetic field to decelerate the capsule endoscope to a predetermined speed when the capsule endoscope approaches the second position,
wherein the variable magnetic field comprises a base magnetic field generated by a second magnet and an induced magnetic field generated by a first induction coil; and the direction of a magnetic axis of the first induction coil is kept in a fixed orientation, and the variable magnetic field is changed by changing at least one of the relative position of a magnetic pole of the second magnet relative to the first magnet, the current magnitude of the first induction coil, and the current direction of the first induction coil.

20. The method according to claim 19, **characterized in** comprising:
controlling the variable magnetic field to accelerate the capsule endoscope when the capsule endoscope is in the first position, and controlling the variable magnetic field to decelerate the capsule endoscope to the predetermined speed when the capsule endoscope approaches the second position, during the movement of the capsule endoscope from the first position to the second position.

21. The method according to claim 19, **characterized in** comprising:
controlling the variable magnetic field to accelerate the capsule endoscope to an initial speed and moving it for a predetermined distance while maintaining the initial speed when the capsule endoscope is in the first position, then controlling the variable magnetic field to decelerate the capsule endoscope to the predetermined speed when the capsule endoscope approaches the second position, during the movement of the capsule endoscope from the first position to the second position.

22. The method according to claim 19, **characterized in** comprising that:
the capsule endoscope shoots the inside of the tissue cavity facing the side of the first position when the capsule endoscope is located in the second position.

23. The method according to claim 19, **characterized in that**:
the first position is a side wall on one side in the tissue cavity, and the second position is a side wall on the other side in the tissue cavity.

24. The method according to claim 19, **characterized in** comprising:
controlling the variable magnetic field to deviate the capsule endoscope to deviating a third position of the capsule endoscope from the first position during the shuttle process,
wherein the process of moving the capsule endoscope from the first position in the tissue cavity to the second position in the tissue cavity and back again to the third position in the tissue cavity is a shuttle process, wherein the first position and the third position are located on one side in the tissue cavity, and the second position is located on the other side in the tissue cavity.

25. The method according to claim 23, **characterized in** comprising:
moving the tissue cavity in a direction forming an included angle with the direction of the magnetic axis to deviate the position of the first magnet from the magnetic axis, and enabling the second magnet to generate a magnetic force acting on the capsule endoscope in the direction of the magnetic axis, when the capsule endoscope is in the first position or the second position in the tissue cavity.

26. A method for controlling movement of a capsule endoscope in a tissue cavity, wherein the capsule endoscope comprise at least a first magnet and an imaging device, **characterized in** comprising:
enabling the capsule endoscope to enter a tissue cavity; and
generating a variable magnetic field for the capsule endoscope, and generating a driving force to the capsule endoscope by controlling the variable magnetic field,
wherein the variable magnetic field comprises a base magnetic field generated by a second magnet and an induction magnetic field generated by a first induction coil; and the direction of a magnetic axis of the first induction coil is kept in a fixed orientation, and the variable magnetic field is changed by changing at least one of the relative position of a magnetic pole of the second magnet relative to the first magnet, the current magnitude of the first induction coil, and the current direction of the first induction coil.

27. The method according to claim 19 or 26, **characterized in that**:
the base magnetic field generates a first magnetic force to the capsule endoscope and the induced magnetic field generates a second magnetic force to the capsule endoscope during the movement of the capsule endoscope from the first position to the second position, whereby generating a deviating movement to the capsule endoscope by forming an included angle between a magnetic force direction of the first magnetic force and a magnetic force direction of the second magnetic force.

28. The method according to claim 19 or 26, **characterized in** comprising:
controlling the variable magnetic field so that the capsule endoscope is on a liquid surface and remains in a position relative to the tissue cavity, and moving the tissue cavity and shooting the inside of it, wherein fluid is contained in the tissue cavity and forms the liquid surface in the tissue cavity.

29. The method according to claim 19 or 26, **characterized in that**:
the first induction coil is arranged on the same side as the second magnet.

30. The method according to claim 19 or 26, **characterized in that**:
the magnetic control device further comprises a second induction coil arranged on a different side from the first induction coil, and the second induction coil is arranged on a different side from the second magnet.

31. The method according to claim 30, **characterized in that**:
the magnetic control device further comprises a third induction coil that generates a magnetic force acting on the capsule endoscope to position the capsule endoscope in a predetermined position within the tissue cavity.

32. The method according to claim 31, **characterized in that**:
the diameter of the third induction coil is smaller than the diameter of the first induction coil and the diameter of the second induction coil.

33. The method according to claim 32, **characterized in that**:
the third induction coil is arranged on the same side as the second induction coil.

34. The method according to claim 19 or 26, **characterized in** comprising:
adjusting the orientation of the imaging device at the other end of the capsule endoscope with one end of the capsule endoscope located on the side wall in the tissue cavity being as a fulcrum by controlling the variable magnetic field, when the capsule endoscope is located on the side wall in the tissue cavity.

35. The method according to claim 19, **characterized in that**:
the direction of the magnetic axis is a vertical direction, and the magnetic axis passes through the second magnet.

36. The method according to claim 19, **characterized in** comprising:
arranging the second magnet around the first induction coil in such a manner that the second magnet is rotatable around a point as the center at which it intersects with the magnetic axis of the first induction coil.

37. The method according to claim 19, **characterized in** comprising:
further moving the capsule endoscope to a fourth position other than the first position and the second position before the capsule endoscope is moved to the second position of the tissue cavity during the shuttle process, wherein the fourth position is not located in a plane formed by the first position, the second position and a third position,
wherein the process of moving the capsule endoscope from the first position of the tissue cavity to the second position of the tissue cavity and back again to the third position of the tissue cavity is a shuttle process, wherein the first position and the third position are located on one side of the tissue cavity, and the second position is located on the other side of the tissue cavity.

38. The method according to claim 37, **characterized in** comprising:
further moving the capsule endoscope to a fifth position other than the second position and the third position before the capsule endoscope is moved to the third position of the tissue cavity during the shuttle process, wherein the fifth position is not located in a plane formed by the first position, the second position and the third position.

39. The method according to claim 38, **characterized in that**:
the first position, the second position, the third position, the fourth position and the fifth position are all located on the inner wall of the tissue cavity.
